# EUROPEAN PATENT SPECIFICATION

(11) **EP 1 988 943 B1**
(45) Date of publication and mention of the grant of the patent: **24.11.2010**
(21) Application number: 07750501.4
(22) Date of filing: 12.02.2007
(51) Int. Cl.: A61L 27/34, A61L 31/10, A61L 27/54, A61L 31/16

(54) **COATING COMPRISING AN ADHESIVE POLYMERIC MATERIAL FOR A MEDICAL DEVICE AND METHOD FOR PREPARING THE SAME**
BESCHICHTUNG MIT POLYMERHAFTMATERIAL FÜR EINE MEDIZINISCHE VORRICHTUNG UND HERSTELLUNGSVERFAHREN DAFÜR
REVÊTEMENT COMPRENANT UN MATÉRIAU POLYMÈRE ADHÉSIF POUR UN DISPOSITIF MÉDICAL ET SON PROCÉDÉ DE PRÉPARATION

(30) Priority: 13.02.2006 US 353625
(43) Date of publication of application: 12.11.2008
(73) Proprietor: Boston Scientific Limited, Hastings Christ Church (BB)
(72) Inventor: KAMATH, Kalpana, R., Natick, MA 01760 (US); PATEL, Hatal, Natick, MA 01760-1537 (US)
(74) Representative: Peterreins, Frank
(86) International application number: PCT/US2007/003670
(87) International publication number: WO 2007/095167

(56) References cited:
- WO-A-2006/055237
- US-A1- 2005 037 047
- US-A1- 2005 220 853
- US-B1- 6 251 136

## Description

### 1. FIELD OF THE INVENTION

This invention relates generally to a medical device having a coating disposed on at least a portion of the surface of the medical device. More particularly, this invention is directed to a coating for a medical device in which the coating comprises an adhesive polymeric material, having a certain degree of surface tack, disposed on at least a portion of the medical device surface. The coating also comprises an outermost coating layer comprising a biologically active material in particulate form disposed on and adhering to at least a portion of the first coating layer. The coating is capable of delivering a biologically active material to a patient. The invention is also directed to a method for manufacturing such a coated medical device.

### 2. BACKGROUND OF THE INVENTION

A variety of medical conditions are treated by introducing an insertable or implantable medical device into the body. Exposure to a medical device which is implanted or inserted into the body of a patient can cause the body tissue to exhibit adverse physiological reactions. For instance, the insertion or implantation of certain catheters or stents can lead to the formation of emboli or clots in blood vessels. Similarly, the implantation of urinary catheters can cause infections, particularly in the urinary tract. Other adverse reactions to medical devices include cell proliferation which can lead to hyperplasia, occlusion of blood vessels, platelet aggregation, rejection of artificial organs, and calcification.

In order to address such adverse effects, medical devices have included biologically active materials. Such materials can be incorporated into the materials used to make the device. Alternatively, the biologically active material(s) can be included in a coating that is applied to a surface of the medical device.

Moreover, medical devices that include a biologically active material can be used for direct or local administration of the biologically active material to a particular part of the patient's body. For instance, stents having coatings that include a biologically active material can be used to treat or prevent restenosis. In some instances, the coating can also include a polymeric material that affects the delivery or release of the biologically active material. For example, various types of coated stents in which the coating includes a biologically active material have been used for localized delivery of drugs to a body lumen. *See, e.g.,* U.S. Patent No. 6,099,562 to Ding et al*.* Such direct or local administration may be more preferred than systemic administration of a biologically active material. Systemic administration requires larger amounts and/or higher concentrations of the biologically active material because of indirect delivery of such materials to the afflicted area. Also, systemic administration may cause side effects which may not be a problem when the biologically active material is locally administered.

Given the advantages of medical devices that include a biologically active material, there exists a need for such devices, particularly medical devices that have a coating comprising a biologically active material and a polymeric material. Of particular interest are medical device coatings that can control the delivery and release kinetics of a biologically active material from the coating and that can be fabricated with minimal efforts. For example, many coatings are made by dissolving a polymer in a solvent and including a biologically active material in the solution. It is important to use a solvent that does not adversely affect the activity of the biologically active material and preferably to use a solvent that can dissolve the biologically active material as well as the polymeric material. Selecting an appropriate solvent that can dissolve both the polymeric material and the biologically active material, without adversely affecting the biologically active material can pose difficulties. Thus, there remains a need for coatings containing a polymeric material and a biologically active material where the biologically active material need not be dissolved in a solvent with the polymeric material.
It is known from US 2005/0220853 a medical device having an adhesive region and a therapeutic agent in powder form applied to the adhesive region surface. **3. SUMMARY OF THE INVENTION**

The present invention is directed to a medical device according to claim 1 The coating can be formed without using a solvent to dissolve the biologically active material. In particular, the invention provides a medical device in which the release rate or kinetics of a biologically active material from the coating can be better controlled with minimal formulation efforts, such as by building alternate layers of polymer only and drug-only layers. Controlling the release rate or kinetics of the biologically active material is desirable. For example, prior to the delivery of the medical device, a minimal release of biologically active material from the coating is desired. Upon implantation of the medical device, there may be a need for a larger amount of biologically active material to be released from the coating. Also, the present invention provides a medical device having a coating suitable for delivery of biologically active material that are susceptible to degradation by solvents, other excipients, process parameters, or drying at elevated temperature. Furthermore, the present invention provides for an efficient and simple method of manufacturing such a medical device coating with reduction or elimination of residual solvents, and excessive handling of active material. The invention also provides a medical device in which the loading of a biologically active material on a device can be achieved with minimal formulation. The invention also provides the benefit of selective deposition of the active material on desired areas of the device, and hence the ability to target the site/location of drug delivery.

The present invention provides a medical device comprising a surface and a coating disposed on at least a portion of the surface, wherein the coating comprises a first layer comprising an adhesive polymeric material having a surface tack of 1 to 25 grams, and an outermost layer adhering to at least a part of the first layer, wherein the outermost layer comprises a first biologically active material in particulate form and wherein the outermost layer is substantially free of any polymeric material. In certain embodiments, the surface tack can be 5 to 20 grams or 10 to 12 grams. Also, the tack of the adhesive polymeric material can be measured by a texture analyzer. The tack of the adhesive polymeric material can be measured by a texture analyzer when the compressive force is 40 grams, when the compressive force time is 5 seconds, when the test speed is 0.25 mm/s and/or when the pulling distance is 2 mm. Furthermore, the adhesive polymeric material can be a biostable polymer or a biodegradable polymer. The biologically active material can comprise paclitaxel, rapamycin, everolimus or tacrolimus. Also, the coating can comprise a second layer disposed under the first layer. The second layer can comprise a second polymeric material, which is adhesive and has a tack of 1 to 25 grams. The second layer can also comprise a second biologically active material. Moreover, the coating can comprise a third coating layer disposed between the first coating layer and the second coating layer; wherein the third coating layer comprises a second biologically active material in particulate form and wherein the third coating layer is substantially free of any polymeric material. In certain embodiments, the medical device can be a stent.

In an alternate embodiment, the invention provides a stent comprising a surface and a coating disposed on at least a portion of the surface. The coating comprises a first layer comprising an adhesive polymeric material having a surface tack of 1 to 25 grams when measured by a texture analyzer when the compressive force is 40 grams, the compressive force time is 5 seconds, the test speed is 0.25 mm/s and the pulling distance is 2 mm. The coating also comprises an outermost layer adhering to the first layer, wherein the outermost layer comprises a first biologically active material in particulate form and wherein the outermost layer is substantially free of any polymeric material.

In another embodiment, the invention provides a method for coating a medical device according to claim 2. The medical device shall have a surface comprising forming a first coating layer comprising an adhesive polymeric material having a surface tack of 1 to 25 grams on a portion of the surface. The medical device shall also have a surface comprising adhering an outermost coating layer comprising a biologically active material in particulate form on at least part of the first coating layer, wherein the outermost layer is substantially free of any polymeric material. In certain embodiments, the surface tack can be 5 to 20 grams or 10 to 12 grams. The outermost coating layer can be formed by spraying the biologically active onto at least part of the first coating layer, dipping the medical device into the biologically active material or by rolling the medical device in the biologically active material. Also, the tack of the adhesive polymeric material can be measured by a texture analyzer. The tack of the adhesive polymeric material can be measured by a texture analyzer when the compressive force is 40 grams, when the compressive force time is 5 seconds, when the test speed is 0.25 mm/s and/or when the pulling distance is 2 mm. Furthermore, the adhesive polymeric material can be a biostable polymer or a biodegradable polymer. The biologically active material can comprise paclitaxel, rapamycin, everolimus or tacrolimus. Also, the method can comprise forming a second layer disposed under the first layer. The second layer can comprise a second polymeric material, which is adhesive and has a tack of 1 to 25 grams. The second layer can also comprise a second biologically active material. Moreover, the method can comprise forming a third coating layer disposed between the first coating layer and the second coating layer. The third coating layer can comprise a second biologically active material in particulate form and can be substantially free of any polymeric material.

### 4. BRIEF DESCRIPTION OF THE DRAWINGS

**Figure 1A** is a cross-sectional view of a coating for a medical device having a first coating layer and an outermost coating layer comprising a biologically active material in particulate form that is disposed over at least a portion of the first coating layer.

**Figure 1B** is a cross-sectional view of a coating for a medical device having a first coating layer and a discontinuous outermost coating layer comprising a biologically active material in particulate form that is disposed over at least a portion of the first coating layer.

**Figure 2** is a cross-sectional view of the coating in **Figure 1A** which further includes a second coating layer disposed under the first coating layer.

**Figure 3** is a cross-sectional view of the coating in **Figure 2** which further includes a third coating layer of a biologically active material in particulate form disposed between the first and second coating layers.

**Figure 4** is a graph summarizing measurements of surface tack versus drying time for a solution of 20 percent by weight (wt%) of a styrene-isobutylene copolymer in toluene as measured by a texture analyzer.

### 5. DETAILED DESCRIPTION

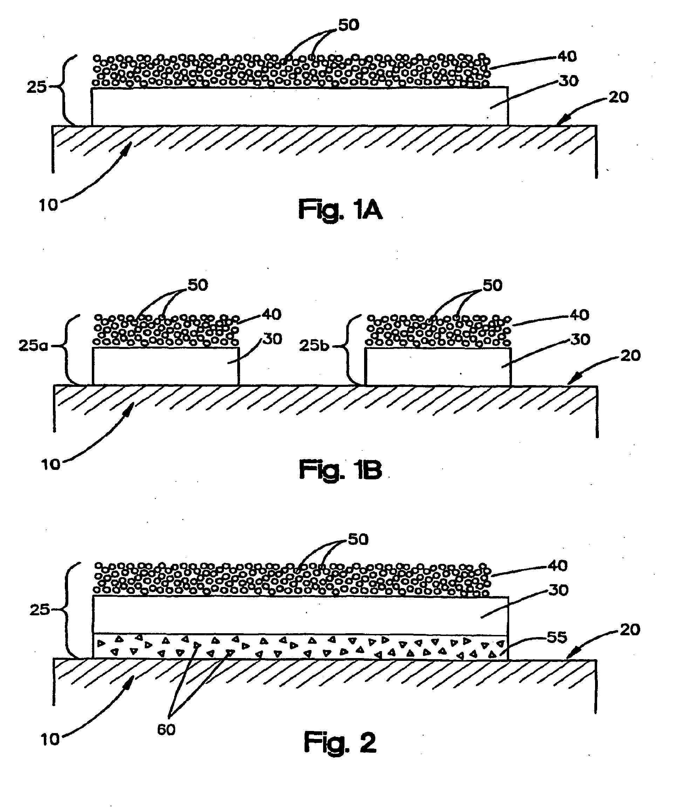

The medical device of the present invention has a surface and a coating disposed on at least a portion of the surface. In one embodiment, the coating comprises a first coating layer and an outermost coating layer disposed upon and adhering to the first coating layer. **Figure 1A** shows such an embodiment of the invention. More specifically, **Figure 1A** is a cross-sectional view of a part of a medical device **10** having a surface **20.** At least a portion of the surface **20** is covered by a coating **25.** The coating **25** comprises a first coating layer **30** which comprises an adhesive polymeric material. This adhesive polymeric material has a surface tack of 1 to 25 grams. Preferably, the surface tack is 5 to 20 grams. More preferably the surface tack is 10 to 12 grams. The surface tack of the adhesive polymeric material is the force required to separate the probe of the texture analyzer from the test surface as it is lifted from the surface. In certain embodiments, the first coating layer **30** can also comprise a biologically active material that can be the same as or different from the biologically active material of the outermost coating layer **40.**

Disposed on at least a part of and adhering to the first coating layer **30** is an outermost coating layer **40** which comprises a biologically active material **50** in particulate form. The outermost layer **40** is substantially free of any polymeric material, *i.e.* contains <1% by weight of a polymeric material, or free of any polymeric material. The surface tack of the adhesive polymeric material in the underlying first coating layer **30** allows the biologically active material **50** to adhere to the first coating layer **30** without the use of a polymeric material in the outermost coating layer **40.** Since the outermost coating layer **40** is substantially free of or free of a polymeric material, the outermost coating layer **40** can be formed without exposing the biologically active material to a solvent needed to dissolve polymeric materials.

**Figure 1B** is a cross-sectional view of another embodiment of a medical device **10** having a surface **20** and a coating disposed upon a portion of the surface. The coating is discontinuous and comprises two regions **25a** and **25b.** Each region **25a, 25b** includes a first coating layer **30** comprising an adhesive polymeric material and an outermost coating layer **40** disposed on at least a part of and adhering to the first coating layer. The outermost coating layer **40** comprises a biologically active material in particulate form **50** and is substantially free or free of a polymeric material.

**Figure 2** is a cross-sectional view of another embodiment of the present invention. In this embodiment a medical device **10** comprises a surface **20.** At least a portion of the surface **20** is coated by a coating **25.** Like the embodiment of **Figure 1A****,** the coating **25** in **Figure 2** comprises a first coating layer **30** comprising an adhesive polymeric material. In some embodiments, the first coating layer **30** can comprise a biologically active material. An outermost coating layer **40** is disposed on at least a part of and adheres to the first coating layer **30.** The outermost coating layer **40** comprises a biologically active material in particulate form **50** and is substantially free of or free of a polymeric material. Unlike the embodiment in **Figure 1A****,** this embodiment includes a second coating layer **55** disposed under the first coating layer **30.** Although **Figure 2** shows the second coating layer being disposed directly under the first coating layer **30,** in other embodiments intervening coating layers can be disposed between the first coating layer **30** and second coating layer **55.** The second coating layer **55** preferably comprises a polymeric material that can but need not have a surface tack of 1 to 25 grams. Also, the second coating layer **55** can include a biologically active material **60** that is the same as or different from the biologically active material of the outermost coating layer **40.**

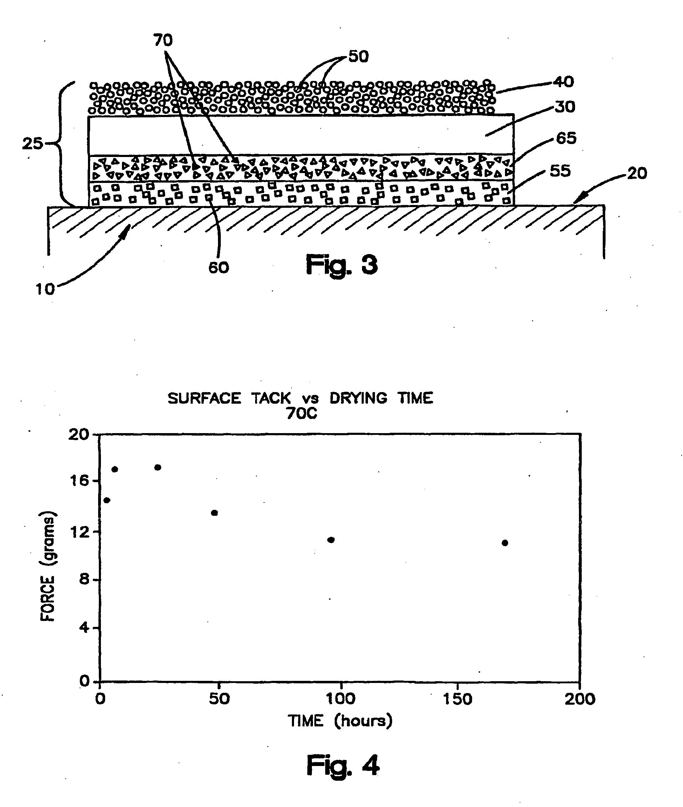

**Figure 3** shows another embodiment in which a medical device **10** comprises a surface **20** having a coating **25** disposed on at least a portion of the surface **20.** Like the embodiment of **Figure 2****,** the coating **25** in **Figure 3** comprises a first coating layer **30** comprising an adhesive polymeric material. An outermost coating layer **40** is disposed on at least a part of and adheres to the first coating layer **30.** The outermost coating layer **40** comprises a biologically active material in particulate form and is substantially free of or free of a polymeric material. A second coating layer **55,** which preferably comprises a polymeric material, is disposed under the first coating layer **30.** The second coating layer **55** can include a biologically active material **60** that is the same as or different from the biologically active material of the outermost coating layer **40.** Unlike the embodiment in **Figure 2****,** this embodiment includes a third coating layer **65** that comprises a biologically active material in particulate form **70** and is substantially free of or free of a polymeric material. The third coating layer **65** is disposed between the first coating layer **30** and the second coating layer **55.**

The above embodiments can include additional coating layers under the layers shown or in between the layers. Some or all of these additional layers can include polymeric material. Alternatively, some or all of the additional layers can include a biologically active material in particulate form and is substantially free or free of a polymeric material.

### A. Suitable Medical Devices

The coated medical devices of the present invention can be inserted and implanted in the body of a patient. Medical devices suitable for the present invention include, but are not limited to, stents, surgical staples, catheters, such as balloon catheters, central venous catheters, and arterial catheters, guidewires, cannulas, cardiac pacemaker leads or lead tips, cardiac defibrillator leads or lead tips, implantable vascular access ports, blood storage bags, blood tubing, vascular or other grafts, intra-aortic balloon pumps, heart valves, cardiovascular sutures, total artificial hearts and ventricular assist pumps, and extra-corporeal devices such as blood oxygenators, blood filters, septal defect devices, hemodialysis units, hemoperfusion units and plasmapheresis units.

Medical devices suitable for the present invention include those that have a tubular or cylindrical-like portion. The tubular portion of the medical device need not be completely cylindrical. For instance, the cross-section of the tubular portion can be any shape, such as rectangle, a triangle, *etc*., not just a circle. Such devices include, without limitation, stents, balloon catheters, and grafts. A bifurcated stent is also included among the medical devices which can be fabricated by the method of the present invention.

Medical devices that are particularly suitable for the present invention include any kind of stent for medical purposes which is known to the skilled artisan. Suitable stents include, for example, vascular stents such as self-expanding stents and balloon expandable stents. Examples of self-expanding stents useful in the present invention are illustrated in U.S. Patent Nos. 4,655,771 and 4,954,126 issued to Wallsten and 5,061,275 issued to Wallsten et al. Examples of appropriate balloon-expandable stents are shown in U.S. Patent No. 5,449,373 issued to Pinchasik et al. In preferred embodiments, the stent suitable for the present invention is an Express stent. More preferably, the Express stent is an Express™ stent or an Express2™ stent (Boston Scientific, Inc. Natick, Mass.).

Medical devices that are suitable for the present invention may be fabricated from metallic, ceramic, or polymeric materials, or a combination thereof. Preferably, the materials are biocompatible. Metallic material is more preferable. Suitable metallic materials include metals and alloys based on titanium (such as nitinol, nickel titanium alloys, thermo-memory alloy materials), stainless steel, tantalum, nickel-chrome, or certain cobalt alloys including cobalt-chromium-nickel alloys such as Elgiloy® and Phynox®. Metallic materials also include clad composite filaments, such as those disclosed in WO 94/16646.

Suitable ceramic materials include, but are not limited to, oxides, carbides, or nitrides of the transition elements such as titanium oxides, hafnium oxides, iridiumoxides, chromium oxides, aluminum oxides, and zirconiumoxides. Silicon based materials, such as silica, may also be used. The polymeric material may be biostable. Also, the polymeric material may be biodegradable. Suitable polymeric materials include, but are not limited to, styrene isobutylene styrene, polyetheroxides, polyvinyl alcohol, polyglycolic acid, polylactic acid, polyamides, poly-2-hydroxy-butyrate, polycaprolactone, poly(lactic-co-clycolic)acid, and Teflon.

Polymeric materials may be used for forming the medical device in the present invention include without limitation isobutylene-based polymers, polystyrene-based polymers, polyacrylates, and polyacrylate derivatives, vinyl acetate-based polymers and its copolymers, polyurethane and its copolymers, silicone and its copolymers, ethylene vinylacetate, polyethylene terephtalate, thermoplastic elastomers, polyvinyl chloride, polyolefins, cellulosics, polyamides, polyesters, polysulfones, polytetrafluorethylenes, polycarbonates, acrylonitrile butadiene styrene copolymers, acrylics, polylactic acid, polyglycolic acid, polycaprolactone, polylactic acid-polyethylene oxide copolymers, cellulose, collagens, and chitins.

Other polymers that are useful as materials for medical devices include without limitation dacron polyester, poly(ethylene terephthalate), polycarbonate, polymethylmethacrylate, polypropylene, polyalkylene oxalates, polyvinylchloride, polyurethanes, polysiloxanes, nylons, poly(dimethyl siloxane), polycyanoacrylates, polyphosphazenes, poly(amino acids), ethylene glycol I dimethacrylate, poly(methyl methacrylate), poly(2-hydroxyethyl methacrylate), polytetrafluoroethylene poly(HEMA), polyhydroxyalkanoates, polytetrafluorethylene, polycarbonate, poly(glycolide-lactide) copolymer, polylactic acid, poly(γ-caprolactone), poly(γ -hydroxybutyrate), polydioxanone, poly(γ -ethyl glutamate), polyiminocarbonates, poly(ortho ester), polyanhydrides, alginate, dextran, chitin, cotton, polyglycolic acid, polyurethane, or derivatized versions thereof, i.e., polymers which have been modified to include, for example, attachment sites or crosslinking groups, *e.g*., RGD, in which the polymers retain their structural integrity while allowing for attachment of cells and molecules, such as proteins, nucleic acids, and the like.

Medical devices may also be made with non-polymeric materials. Examples of useful non-polymeric materials include sterols such as cholesterol, stigmasterol, β-sitosterol, and estradiol; cholesteryl esters such as cholesteryl stearate; C₁₂ -C₂₄ fatty acids such as lauric acid, myristic acid, palmitic acid, stearic acid, arachidic acid, behenic acid, and lignoceric acid; C₁₈ -C₃₆ mono-, di- and triacylglycerides such as glyceryl monooleate, glyceryl monolinoleate, glyceryl monolaurate, glyceryl monodocosanoate, glyceryl monomyristate, glyceryl monodicenoate, glyceryl dipalmitate, glyceryl didocosanoate, glyceryl dimyristate, glyceryl didecenoate, glyceryl tridocosanoate, glyceryl trimyristate, glyceryl tridecenoate, glycerol tristearate and mixtures thereof; sucrose fatty acid esters such as sucrose distearate and sucrose palmitate; sorbitan fatty acid esters such as sorbitan monostearate, sorbitan monopalmitate and sorbitan tristearate; C₁₆ -C₁₈ fatty alcohols such as cetyl alcohol, myristyl alcohol, stearyl alcohol, and cetostearyl alcohol; esters of fatty alcohols and fatty acids such as cetyl palmitate and cetearyl palmitate; anhydrides of fatty acids such as stearic anhydride; phospholipids including phosphatidylcholine (lecithin), phosphatidylserine, phosphatidylethanolamine, phosphatidylinositol, and lysoderivatives thereof; sphingosine and derivatives thereof; sphingomyelins such as stearyl, palmitoyl, and tricosanyl sphingomyelins; ceramides such as stearyl and palmitoyl ceramides; glycosphingolipids; lanolin and lanolin alcohols; and combinations and mixtures thereof. Preferred non-polymeric materials include cholesterol, glyceryl monostearate, glycerol tristearate, stearic acid, stearic anhydride, glyceryl monooleate, glyceryl monolinoleate, and acetylated monoglycerides.

### B. Suitable Biologically Active Materials

The coatings of the present invention comprise one or more biologically active materials. In particular, the outermost coating layer comprises a biologically active material in particulate form. The term "therapeutic agent" as used in the present invention encompasses drugs, genetic materials, and biological materials and can be used interchangeably with "biologically active material". Non-limiting examples of suitable therapeutic agent include heparin, heparin derivatives, urokinase, dextrophenylalanine proline arginine chloromethylketone (PPack), enoxaprin, angiopeptin, hirudin, acetylsalicylic acid, tacrolimus, everolimus, rapamycin (sirolimus), pimecrolimus, amlodipine, doxazosin, glucocorticoids, betamethasone, dexamethasone, prednisolone, corticosterone, budesonide, sulfasalazine, rosiglitazone, mycophenolic acid, mesalamine, paclitaxel (as well as its derivatives, analogs or paclitaxel bound to proteins, *e.g*. Abraxane™), 5-fluorouracil, cisplatin, vinblastine, vincristine, epothilones, methotrexate, azathioprine, adriamycin, mutamycin, endostatin, angiostatin, thymidine kinase inhibitors, cladribine, lidocaine, bupivacaine, ropivacaine, D-Phe-Pro-Arg chloromethyl ketone, platelet receptor antagonists, anti-thrombin antibodies, anti-platelet receptor antibodies, aspirin, dipyridamole, protamine, hirudin, prostaglandin inhibitors, platelet inhibitors, trapidil, liprostin, tick antiplatelet peptides, 5-azacytidine, vascular endothelial growth factors, growth factor receptors, transcriptional activators, translational promoters, antiproliferative agents, growth factor inhibitors, growth factor receptor antagonists, transcriptional repressors, translational repressors, replication inhibitors, matrix metalloproteinase inhibitors, inhibitory antibodies, antibodies directed against growth factors, bifunctional molecules consisting of a growth factor and a cytotoxin, bifunctional molecules consisting of an antibody and a cytotoxin, cholesterol lowering agents, vasodilating agents, agents which interfere with endogenous vasoactive mechanisms, antioxidants, probucol, antibiotic agents, penicillin, cefoxitin, oxacillin, tobranycin, angiogenic substances, fibroblast growth factors, estrogen, estradiol (E2), estriol (E3), 17-beta estradiol, digoxin, beta blockers, captopril, enalopril, statins, steroids, vitamins, paclitaxel, 2'-succinyl-taxol, 2'-succinyl-taxol triethanolamine, 2'-glutaryl-taxol, 2'-glutaryl-taxol triethanolamine salt, 2'-O-ester with N-(dimethylaminoethyl) glutamine, 2'-O-ester with N-(dimethylaminoethyl) glutamide hydrochloride salt, nitroglycerin, nitrous oxides, nitric oxides, antibiotics, aspirins, digitalis, estrogen, estradiol and glycosides. In one embodiment, the therapeutic agent is a smooth muscle cell inhibitor or antibiotic. In a preferred embodiment, the therapeutic agent is taxol (*e.g*., Taxol®), or its analogs or derivatives. In another preferred embodiment, the therapeutic agent is paclitaxel, or its analogs or derivatives. In yet another preferred embodiment, the therapeutic agent is an antibiotic such as erythromycin, amphotericin, rapamycin, adriamycin, etc.

The term "genetic materials" means DNA or RNA, including, without limitation, of DNA/RNA encoding a useful protein stated below, intended to be inserted into a human body including viral vectors and non-viral vectors.

The term "biological materials" include cells, yeasts, bacteria, proteins, peptides, cytokines and hormones. Examples for peptides and proteins include vascular endothelial growth factor (VEGF), transforming growth factor (TGF), fibroblast growth factor (FGF), epidermal growth factor (EGF), cartilage growth factor (CGF), nerve growth factor (NGF), keratinocyte growth factor (KGF), skeletal growth factor (SGF), osteoblast-derived growth factor (BDGF), hepatocyte growth factor (HGF), insulin-like growth factor (IGF), cytokine growth factors (CGF), platelet-derived growth factor (PDGF), hypoxia inducible factor-1 (HIF-1), stem cell derived factor (SDF), stem cell factor (SCF), endothelial cell growth supplement (ECGS), granulocyte macrophage colony stimulating factor (GM-CSF), growth differentiation factor (GDF), integrin modulating factor (IMF), calmodulin (CaM), thymidine kinase (TK), tumor necrosis factor (TNF), growth hormone (GH), bone morphogenic protein (BMP) (*e.g*., BMP-2, BMP-3, BMP-4, BMP-5, BMP-6 (Vgr-1), BMP-7 (PO-1), BMP-8, BMP-9, BMP-10, BMP-11, BMP-12, BMP-14, BMP-15, BMP-16, etc.), matrix metalloproteinase (MMP), tissue inhibitor of matrix metalloproteinase (TIMP), cytokines, interleukin (*e.g*., IL-1, IL-2, IL-3, IL-4, IL-5, IL-6, IL-7, IL-8, IL-9, IL-10, IL-11, IL-12, IL-15, etc.), lymphokines, interferon, integrin, collagen (all types), elastin, fibrillins, fibronectin, vitronectin, laminin, glycosaminoglycans, proteoglycans, transferrin, cytotactin, cell binding domains (*e.g*., RGD), and tenascin. Currently preferred BMP's are BMP-2, BMP-3, BMP-4, BMP-5, BMP-6, BMP-7. These dimeric proteins can be provided as homodimers, heterodimers, or combinations thereof, alone or together with other molecules. Cells can be of human origin (autologous or allogeneic) or from an animal source (xenogeneic), genetically engineered, if desired, to deliver proteins of interest at the transplant site. The delivery media can be formulated as needed to maintain cell function and viability. Cells include progenitor cells (*e.g*., endothelial progenitor cells), stem cells (*e.g*., mesenchymal, hematopoietic, neuronal); stromal cells, parenchymal cells, undifferentiated cells, fibroblasts, macrophage, and satellite cells.
Other non-genetic therapeutic agents include:
- anti-thrombogenic agents such as heparin, heparin derivatives, hirudin, urokinase, and PPack (dextrophenylalanine proline arginine chloromethylketone);
- anti-proliferative agents such as enoxaprin, angiopeptin, or monoclonal antibodies capable of blocking smooth muscle cell proliferation, tacrolimus, everolimus, amlodipine and doxazosin;
- anti-inflammatory agents such as glucocorticoids, betamethasone, dexamethasone, prednisolone, corticosterone, biophosphonates, cell adhesion molecules, acetylsalicylic acid, budesonide, estrogen, sulfasalazine, rosiglitazone, mycophenolic acid and mesalamine;

- anti-neoplastic/anti-proliferative/anti-miotic agents such as paclitaxel, 5-fluorouracil, cisplatin, vinblastine, vincristine, epothilones, methotrexate, azathioprine, adriamycin and mutamycin; endostatin, angiostatin and thymidine kinase inhibitors, cladribine, taxol and its analogs or derivatives;
- anesthetic agents such as lidocaine, bupivacaine, and ropivacaine;
- anti-coagulants such as D-Phe-Pro-Arg chloromethyl ketone, an RGD peptide-containing compound, heparin, antithrombin compounds, platelet receptor antagonists, anti-thrombin antibodies, anti-platelet receptor antibodies, aspirin (aspirin is also classified as an analgesic, antipyretic and anti-inflammatory drug), dipyridamole, protamine, hirudin, prostaglandin inhibitors, platelet inhibitors, antiplatelet agents such as trapidil or liprostin and tick antiplatelet peptides;
- DNA demethylating drugs such as 5-azacytidine, which is also categorized as a RNA or DNA metabolite that inhibit cell growth and induce apoptosis in certain cancer cells;
- vascular cell growth promoters such as growth factors, vascular endothelial growth factors (VEGF, all types including VEGF-2), growth factor receptors, transcriptional activators, and translational promoters;
- vascular cell growth inhibitors such as anti-proliferative agents, growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin;
- cholesterol-lowering agents such as HMG-CoA reductase inhibitors, vasodilating agents, and agents which interfere with endogenous vasoactive mechanisms;
- anti-oxidants, such as probucol;
- antibiotic agents, such as penicillin, cefoxitin, oxacillin, tobranycin, rapamycin (sirolimus);
- angiogenic substances, such as acidic and basic fibroblast growth factors, estrogen including estradiol (E2), estriol (E3) and 17-beta estradiol;
- drugs for heart failure, such as digoxin, beta-blockers, angiotensin-converting enzyme (ACE) inhibitors including captopril and enalopril, statins and related compounds;
- macrolides such as sirolimus or everolimus; and
- matrix metalloproteinase inhibitors such as Batimastat.

Preferred biological materials include anti-proliferative drugs such as steroids, vitamins, and restenosis-inhibiting agents. Preferred restenosis-inhibiting agents include microtubule stabilizing agents such as Taxol®, paclitaxel (*i.e*., paclitaxel, paclitaxel analogs, or paclitaxel derivatives, and mixtures thereof). For example, derivatives suitable for use in the present invention include 2'-succinyl-taxol, 2'-succinyl-taxol triethanolamine, 2'-glutaryl-taxol, 2'-glutaryl-taxol triethanolamine salt, 2'-O-ester with N-(dimetllylaminoethyl) glutamine, and 2'-O-ester with N-(dimethylaminoethyl) glutamide hydrochloride salt.

Other suitable therapeutic agents include tacrolimus; halofuginone; inhibitors of HSP90 heat shock proteins such as geldanamycin; microtubule stabilizing agents such as epothilone D; phosphodiesterase inhibitors such as cliostazole; Barkct inhibitors; phospholamban inhibitors; and Serca 2 gene/proteins.

Other preferred therapeutic agents include nitroglycerin, nitrous oxides, nitric oxides, aspirins, digitalis, estrogen derivatives such as estradiol and glycosides.

In one embodiment, the therapeutic agent is capable of altering the cellular metabolism or inhibiting a cell activity, such as protein synthesis, DNA synthesis, spindle fiber formation, cellular proliferation, cell migration, microtubule formation, microfilament formation, extracellular matrix synthesis, extracellular matrix secretion, or increase in cell volume. In another embodiment, the therapeutic agent is capable of inhibiting cell proliferation and/or migration.

In certain embodiments, the therapeutic agents for use in the medical devices of the present invention can be synthesized by methods well known to one skilled in the art. Alternatively, the therapeutic agents can be purchased from chemical and pharmaceutical companies.

### C. Suitable Polymeric Materials

The polymeric materials useful for the coating of the present invention should be ones that are biocompatible and avoid irritation to body tissue. The polymeric materials can be either biostable or bioabsorbable. Suitable polymeric materials include those discussed above that are used to fabricate medical devices. Also, the polymeric materials useful for the coating of the present invention include without limitation: polyurethanes, silicones (*e.g*., polysiloxanes and substituted polysiloxanes), and polyesters. Also preferable as a polymeric material are styrene-isobutylene-styrene copolymers. Other polymers which can be used include ones that can be dissolved and cured or polymerized on the medical device or polymers having relatively low melting points that can be blended with biologically active materials. Additional suitable polymers include, thermoplastic elastomers in general, polyolefins, polyisobutylene, ethylene-alphaolefin copolymers, acrylic polymers and copolymers, vinyl halide polymers and copolymers such as polyvinyl chloride, polyvinyl ethers such as polyvinyl methyl ether, polyvinylidene halides such as polyvinylidene fluoride and polyvinylidene chloride, polyacrylonitrile, polyvinyl ketones, polyvinyl aromatics such as polystyrene, polyvinyl esters such as polyvinyl acetate, copolymers of vinyl monomers, copolymers of vinyl monomers and olefins such as ethylene-methyl methacrylate copolymers, acrylonitrile-styrene copolymers, ABS
(acrylonitrile-butadiene-styrene) resins, ethylene-vinyl acetate copolymers, polyamides such as Nylon 66 and polycaprolactone, alkyd resins, polycarbonates, polyoxymethylenes, polyimides, polyethers, epoxy resins, rayon-triacetate, cellulose, cellulose acetate, cellulose butyrate, cellulose acetate butyrate, cellophane, cellulose nitrate, cellulose propionate, cellulose ethers, carboxymethyl cellulose, collagens, chitins, polylactic acid, polyglycolic acid, polylactic acid-polyethylene oxide copolymers, EPDM (ethylene-propylene-diene) rubbers, fluorosilicones, polyethylene glycol, polysaccharides, phospholipids, and combinations of the foregoing.

Preferably, for medical devices which undergo mechanical challenges, *e.g.,* expansion and contraction, polymeric materials should be selected from elastomeric polymers such as silicones (*e.g*., polysiloxanes and substituted polysiloxanes), polyurethanes, thermoplastic elastomers, ethylene vinyl acetate copolymers, polyolefin elastomers, and EPDM rubbers. Because of the elastic nature of these polymers, the coating composition is capable of undergoing deformation under the yield point when the device is subjected to forces, stress or mechanical challenge.

The polymeric materials used to form the coating layer underlying should be an adhesive polymeric material having a certain degree of surface tack. Such adhesive polymeric materials should have a surface tack of 1 to 25 grams, preferably 5 to 20 grams and more preferably 10 to 12 grams. The surface tack of a polymeric material is generally measured as the force required to separate a probe or object placed in contact with the surface of a film of the polymeric material. Methods are available to persons of skill in the art to measure the surface tack of a polymeric material. For instance, the surface tack of a polymeric material may be measured by a texture analyzer. An example of a texture analyzer is the Model TA-XT2i manufactured by Texture Technology Corporation.

This texture analyzer is equipped with a probe. The tip of the probe comes into contact with the surface of a film of polymeric material. The surface tack is measured as the force required to separate the tip of the probe from the surface of the film. The measurement of the surface tack by a texture analyzer generally involves four (4) parameters. One of the parameters is the compressive force, which is force which the probe applies to the test surface. This parameter is measured in grams. Another parameter is the compressive force time, which is the time that the compressive force is applied to the test surface. The test speed, which is the speed at which the probe is removed or separated from the test surface is also a parameter. In addition, another parameter is the pulling distance, which is the distance that the probe travels after being removed from the test surface.

Examples of preferred adhesive polymeric materials include without limitation, copolymers of styrene and isobutylene, cyanoacrylate or ethylene vinyl acetate, isobutylene-based polymers, acrylate-based polymers, fibrin or combinations thereof.

### D. Methods for Making the Coatings

In one method of forming the aforementioned coating layers, a coating composition is applied to the surface. Coating compositions can be applied by any method to a surface of a medical device to form a coating layer. Examples of suitable methods include, but are not limited to, spraying such as by conventional nozzle or ultrasonic nozzle, dipping, rolling, electrostatic deposition, ink-jet coating and a batch process such as air suspension, pancoating or ultrasonic mist spraying. Also, more than one coating method can be used to apply a coating composition onto the surface of the medical device.

In the present invention, the coating layer underlying the outermost layer, such as the first coating layer which comprises an adhesive polymeric material, can be prepared by forming a coating composition comprising a polymeric material dissolved or suspended in a solvent. The coating composition can also include a biologically active material. Solvents that may be used to prepare coating compositions include ones which can dissolve or suspend the polymeric material in solution. Examples of suitable solvents include, but are not limited to, tetrahydrofuran, methylethylketone, chloroform, toluene, acetone, isooctane, 1,1,1,-trichloroethane, dichloromethane, isopropanol, IPA, and mixture thereof.

To form the outermost coating layer, a biologically active material in particulate form is applied to the first coating layer, which comprises the adhesive polymeric material. The biologically active material can be sprayed onto the first coating layer. Also, the biologically active material can be applied to the first coating layer by rolling or dipping the medical device surface in the biologically active material. The biologically active material can also be pre-formulated in the form of nanoparticles, microspheres, liposomes, micronized particles, nanocrystals and applied to the first coating layer. Because of adhesive nature of the polymeric material the biologically active material of the outermost layer adheres to the first coating layer. Preferably no solvent is used to form the outermost coating layer. Also, the biologically active material may have an average particle size of less than 10 microns, less than 15 microns, less than 30 microns, less than 60 microns, less than 100 microns, or at least 100 microns.

### 6. EXAMPLES

### Example 1

A composition containing 20 weight percent (wt %) of a styrene-isobutylene copolymer in toluene was prepared. Aluminum coupons were coated with this composition by dipping the coupons into the composition. The speed at which the coupons were removed from the composition was varied according to Table 1. The coated aluminum coupons were dried for three hours at a drying temperature of 70°C. The surface tack of the styrene-isobutylene copolymer was measured by a texture analyzer (Model TA-XT2i made by Texture Technology Corporation). The surface tack was measured as the force required to release (de-bond) the probe of the texture analyzer from the test surface as the probe travels in the upward direction. The texture analyzer was set with the following parameters:
Compressive Force: 40 grams
Compressive Force Time: Time: 5 seconds
Test Speed: 0.25mm/second
Pulling Distance: 2.0mm
Table 1 shows that average force required to separate the probe from the surface of the test films.

**Table 1**

| Sample# | 1 | 2 | 3 | 4 | 5 | 6 |
|---|---|---|---|---|---|---|
| Dip Speed (mm/min) | 12.7 | 20 | 30 | 40 | 50 | 100 |
| Average Force (grams) Required To Separate Probe From Test Surface | 10.55 | 10.59 | 7.70 | 10.41 | 9.17 | 10.55 |
| Std. Dev. | 2.61 | 1.85 | 0.93 | 2.08 | 2.54 | 3.72 |

### Example 2

Samples were prepared according to Example 1. However, the dip speed was varied as shown in Table 2. Also, the drying time for the samples was 4 days instead of 3 hours. Table 2 shows that average force required to separate the probe from the surface of the test films.

**Table 2**

| Sample# | 1 | 2 | 3 | 4 | 5 | 6 | 7 | 8 |
|---|---|---|---|---|---|---|---|---|
| Dip Speed (mm/min) | 12.7 | 12.7 | 20 | 20 | 50 | 50 | 100 | 100 |
| Average Force (grams) Required To Separate Probe From Test Surface | 22.74 | 20.72 | 31.01 | 35.61 | 41.85 | 31.99 | 31.06 | 31.51 |
| Std. Dev. | 3.67 | 1.79 | 4.14 | 4.09 | 7.25 | 12.25 | 6.31 | 4.08 |

### Example 3

Samples were prepared according to Example 1. However, the drying time was varied as shown in Table 3. Also, Table 3 shows that average force required to separate the probe from the surface of the test films for each drying time. Furthermore, **Figure 4** is a graph of surface tack versus drying time. Up to 24 hours of drying time there was a slight increase in the surface tack. The surface tack began to decrease and started leveling off around 11 grams of force after 96 hours of drying time. The surface tack remained around 11 grams of force after 168 hours of drying time. B

**Table 3**

| Time (hours) | Average Force Required To Separate Probe From Test Surface (grams) |
|---|---|
| 3 | 14.5 |
| 6 | 16.99 |
| 24 | 17.16 |
| 48 | 13.6 |
| 96 | 11.67 |
| 168 | 11.18 |

### Example 4

Samples were prepared according to Example 1. However, the amount of styrene in the styrene-isobutylene copolymer used to form the composition was varied as shown in Table 4. Two samples for each composition were prepared and tested. Table 4 summarizes the surface tack results:

**Table 4**

| Batch# | A | | B | | C | | D | | E | |
|---|---|---|---|---|---|---|---|---|---|---|
| Mol % styrene in copolymer | 16.5 | | 16.8 | | 16.3 | | 22.8 | | 21.1 | |
| Experiment # | 1 | 2 | 1 | 2 | 1 | 2 | 1 | 2 | 1 | 2 |
| Average Force (grams) Required To Separate Probe From Test Surface | 21.67 | 16.62 | 23.83 | 23.92 | 6.75 | 5.49 | 3.42 | 1.64 | 3.95 | 4.51 |
| Std. Dev. | 3.36 | 3.51 | 8.36 | 6.03 | 3.23 | 2.55 | 1.85 | 1.80 | 1.47 | 2.03 |

As shown above, the three compositions with approximately 16 mol% styrene in the copolymer have higher surface tack than the two batches with over 20 mol% styrene in the copolymer.

## Claims

1. A medical device (10) comprising a surface (20) and a coating (25) disposed on at least a portion of the surface (20); wherein the coating (25) comprises
(a) a first layer (30) comprising an adhesive polymeric material having a surface tack of 1 to 25 grams when measured by a texture analyzer with compressive force 40 grams, compressive force time 5 seconds, test speed 0.25 mm/s and pulling distance 2 mm; and
(b) an outermost layer (40) adhering to at least a part of the first layer (30), wherein the outermost layer (40) comprises a first therapeutic agent in particulate form (50) and wherein the outermost layer (40) is free of any polymeric material;
wherein the first layer (30) further comprises a therapeutic agent that is the same or different from the biologically active material of the outermost coating layer (40).

2. A method for coating a medical device (10) having surface (20) comprising:
(a) forming a first coating layer (30) comprising an adhesive polymeric material having, on a portion of the surface (20), a surface tack of 1 to 25 grams when measured by a texture analyzer with compressive force 40 grams, compressive three time 5 seconds, test speed 0.25 mm/s and pulling distance 2 mm; and (b) adhering an outermost coating layer (40) comprising a therapeutic agent in particulate form (50) on at least part of the first coating layer (30), wherein the outermost layer (40) is free of any polymeric material:
wherein the first layer (30) further comprises a therapeutic agent that is the same or different from the biologically active material of the outermost coating layer (40).

3. The device of claim 1 or the method of claim 2, wherein the surface tack is 5 to 20 grams.

4. The device or the method of claim 3, wherein the surface tack is 10 to 12 grams.

5. The medical device of claim 1, wherein the adhesive polymeric material is a biostable polymer.

6. The medical device of claim 1, wherein the adhesive polymeric material is a biodegradable polymer.

7. The medical device of claim 1 or the method of claim 2, wherein the therapeutic agent comprises paclitaxel.

8. The medical device of claim 1 or the method of claim 2, wherein the therapeutic agent comprises rapamycin, everolimus or tacrolimus.

9. The medical device of claim 1, wherein the coating comprises a second layer (55) disposed under the first layer (30).

10. The method of claim 2, further comprising forming a second layer (55) on the surface before step (a).

11. The medical device of claim 9 or the method of claim 10, wherein the second layer (55) comprises a second polymeric material.

12. The medical device or the method of claim 11, wherein the second polymeric material is adhesive and has a tack of I to 25 grams when measured by a texture analyzer with compressive force 40 grams, compressive force time 5 seconds, test speed 0.25 mm/s and pulling distance 2 mm; or wherein the second layer (55) comprises a second therapeutic agent (60).

13. The medical device of claim 9, further comprising a third coating layer (65) disposed between the first coating layer (30) and the second coating layer (55); wherein the third coating layer (65) comprises a second therapeutic agent in particulate form (70) and wherein the third coating layer (65) is free of any polymeric material.

14. The medical device of claim 1, wherein the medical device is a stent.

15. The method of claim 2, wherein step (b) is formed by spraying the biologically active onto at least part of the first coating layer (30) or by dipping the medical device into the biologically active material or by rolling the medical device in the biologically active material.

16. The method of claim 10, further comprising terming a third layer (65) disposed between the first coating layer (30) and this second coating layer (55), wherein the third coating layer (65) comprises a second biologically active material in particulate form (70) and wherein the third coating layer (65) is free of any polymeric material.

## Patentansprüche

1. Medizinische Vorrichtung (10), umfassend eine Oberfläche (20) und eine Beschichtung (25), die auf mindestens einem Bereich der Oberfläche (20) angeordnet ist; wobei die Beschichtung (25) umfasst
(a) eine erste Schicht (30), umfassend ein haftendes polymerisches Material mit einer Oberflächenanhaftung von 1 bis 25 Gramm, wenn von einem Strukturanalysator gemessen mit Druckkraft 40 Gramm, Druckkraftzeit 5 Sekunden, Testgeschwindigkeit 0,25 mm/s und Ziehabstand 2 mm; und
(b) eine äußerste Schicht (40), die an mindestens einem Teil der ersten Schicht (30) haftet, wobei die äußerste Schicht (40) einen ersten therapeutischen Wirkstoff in partikelförmiger Form (50) umfasst und wobei die äußerste Schicht (40) frei von jeglichem polymerischen Material ist;
wobei die erste Schicht (30) weiterhin einen therapeutischen Wirkstoff umfasst, der der gleiche ist wie oder verschieden ist von dem biologisch aktiven Material der äußersten Beschichtungsschicht (40).

2. Verfahren zum Beschichten einer medizinischen Vorrichtung (10) mit Oberfläche (20) umfassend:
(a) Bilden einer ersten Beschichtungsschicht (30), umfassend ein haftendes polymerisches Material mit, auf einem Bereich der Oberfläche (20), einer Oberflächenanhaftung von 1 bis 25 Gramm, wenn von einem Strukturanalysator gemessen mit Druckkraft 40 Gramm, Druckkraftzeit 5 Sekunden, Testgeschwindigkeit 0,25 mm/s und Ziehabstand 2 mm; und
(b) Anhaften einer äußersten Beschichtungsschicht (40), umfassend einen therapeutischen Wirkstoff in partikelförmiger Form (50) auf mindestens einem Teil der ersten Beschichtungsschicht (30), wobei die äußerste Schicht (40) frei von jeglichem polymerischen Material ist;
wobei die erste Schicht (30) weiterhin einen therapeutischen Wirkstoff umfasst, der der gleiche ist wie oder verschieden ist von dem biologisch aktiven Material der äußersten Beschichtungsschicht (40).

3. Vorrichtung nach Anspruch 1 oder Verfahren nach Anspruch 2, wobei die Oberflächenanhaftung 5 bis 20 Gramm ist.

4. Vorrichtung oder Verfahren nach Anspruch 3, wobei die Oberflächenanhaftung 10 bis 12 Gramm ist.

5. Medizinische Vorrichtung nach Anspruch 1, wobei das haftende polymerische Material ein biostabiles Polymer ist.

6. Medizinische Vorrichtung nach Anspruch 1, wobei das haftende polymerische Material ein bioabbaubares Polymer ist.

7. Medizinische Vorrichtung nach Anspruch 1 oder Verfahren nach Anspruch 2, wobei der therapeutische Wirkstoff Paclitaxel umfasst.

8. Medizinische Vorrichtung nach Anspruch 1 oder Verfahren nach Anspruch 2, wobei der therapeutische Wirkstoff Rapamycin, Everolimus oder Tacrolimus umfasst.

9. Medizinische Vorrichtung nach Anspruch 1, wobei die Beschichtung eine zweite Schicht (55) umfasst, die unter der ersten Schicht (30) angeordnet ist.

10. Verfahren nach Anspruch 2, weiterhin umfassend Bilden einer zweiten Schicht (55) auf der Oberfläche vor dem Schritt (a).

11. Medizinische Vorrichtung nach Anspruch 9 oder Verfahren nach Anspruch 10, wobei die zweite Schicht (55) ein zweites polymerisches Material umfasst.

12. Medizinische Vorrichtung oder Verfahren nach Anspruch 11, wobei das zweite polymerische Material haftend ist und eine Anhaftung von 1 bis 25 Gramm aufweist, wenn von einem Strukturanalysator gemessen mit Druckkraft 40 Gramm, Druckkraftzeit 5 Sekunden, Testgeschwindigkeit 0,25 mm/s und Ziehabstand 2 mm;
oder wobei die zweite Schicht (55) einen zweiten therapeutischen Wirkstoff (60) umfasst.

13. Medizinische Vorrichtung nach Anspruch 9, weiterhin umfassend eine dritte Beschichtungsschicht (65), die zwischen der ersten Beschichtungsschicht (30) und der zweiten Beschichtungsschicht (55) angeordnet ist; wobei die dritte Beschichtungsschicht (65) einen zweiten therapeutischen Wirkstoff in partikelförmiger Form (70) umfasst und wobei die dritte Beschichtungsschicht (65) frei von jeglichem polymerischen Material ist.

14. Medizinische Vorrichtung nach Anspruch 1, wobei die medizinische Vorrichtung ein Stent ist.

15. Verfahren nach Anspruch 2, wobei der Schritt (b) gebildet wird durch Sprühen des biologisch aktiven auf mindestens einen Teil der ersten Beschichtungsschicht (30) oder durch Eintauchen der medizinischen Vorrichtung in das biologisch aktive Material oder durch Rollen der medizinischen Vorrichtung in dem biologisch aktiven Material.

16. Verfahren nach Anspruch 10, weiterhin umfassend Bilden einer dritten Schicht (65), die zwischen der ersten Beschichtungsschicht (30) und der zweiten Beschichtungsschicht (55) angeordnet ist, wobei die dritte Beschichtungsschicht (65) ein zweites biologisch aktives Material in partikelförmiger Form (70) umfasst und wobei die dritte Beschichtungsschicht (65) frei von jeglichem polymerischen Material ist.

## Revendications

1. Dispositif médical (10) comportant une surface (20) et un revêtement (25) disposé sur au moins une portion de la surface (20), **caractérisé en ce que** le revêtement (25) comporte :
(a) une première couche (30) comprenant un matériau polymère adhésif ayant une adhésivité de surface de 1 à 25 grammes lorsqu'elle est mesurée par un analyseur de texture avec une force de compression de 40 grammes, une durée de force de compression de 5 secondes, une vitesse de test de 0.25 mm/s et une distance de traction de 2 mm ; et
(b) une couche la plus externe (40) qui adhère au moins partiellement à la première couche (30), dans lequel la couche la plus externe (40) comprend un premier agent thérapeutique d'une forme particulière (50) et **en ce que** la couche la plus externe (40) est dépourvue de tout matériau polymère ; dans lequel la première couche (30) comprend de plus un agent thérapeutique qui est identique ou différent du matériau biologiquement actif de la couche de revêtement la plus externe (40).

2. Procédé pour revêtir un dispositif médical (10) ayant une surface (20), comportant :
(a) la formation d'une première couche de revêtement (30) comportant un matériau polymère adhésif ayant, sur une portion de surface (20), une adhésivité de surface de 1 à 25 grammes lorsqu'elle est mesurée par un analyseur de texture ayant une force de compression de 40 grammes, une durée de force de compression de 5 secondes, une vitesse de test de 0.25 mm/s et une distance de traction de 2 mm ; et (b) l'adhérence d'une couche de revêtement la plus externe (40) comportant un agent thérapeutique de forme particulière (50) sur au moins une partie de la première couche de revêtement (30), dans lequel la couche la plus externe (40) est dépourvue de tout matériau polymère ;
dans lequel la première couche (30) comprend de plus un agent thérapeutique qui est identique ou différent du matériau biologiquement actif de la couche (40) de revêtement la plus externe.

3. Dispositif selon la revendication 1 ou procédé selon la revendication 2, **caractérisé en ce que** l'adhésivité de surface est de 5 à 20 grammes.

4. Dispositif ou procédé selon la revendication 3, **caractérisé en ce que** l'adhésivité de surface est de 10 à 12 grammes.

5. Dispositif médical selon la revendication 1, **caractérisé en ce que** le matériau polymère adhésif est un polymère biostable.

6. Dispositif médical selon la revendication 1 ou procédé selon la revendication 2, **caractérisé en ce que** le matériau polymère adhésif est un polymère biodégradable.

7. Dispositif médical selon la revendication 1 ou procédé selon la revendication 2, **caractérisé en ce que** l'agent thérapeutique comprend le paclitaxel.

8. Dispositif médical selon la revendication 1 ou procédé selon la revendication 2, **caractérisé en ce que** l'agent thérapeutique comprend la rapamycine, l'evelimus ou le tacrolimus.

9. Dispositif médical selon la revendication 1, **caractérisé en ce que** le revêtement comporte une deuxième couche (55) disposée dessous la première couche (30).

10. Procédé selon la revendication 2, comportant de plus la formation d'une seconde couche (55) sur la surface avant l'étape (a).

11. Dispositif médical selon la revendication 9 ou procédé selon la revendication 10, **caractérisé en ce que** la deuxième couche (5) comporte un second matériau polymère.

12. Dispositif médical ou procédé selon la revendication 11, **caractérisé en ce que** le second matériau polymère est adhésif et a une adhésivité de 1 à 25 grammes lorsqu'elle est mesurée par un analyseur de texture ayant une force de compression de 40 grammes, une durée de force de compression de 5 secondes, une vitesse de test de 0.25 mm/s et une distance de traction de 2 mm ;
ou **en ce que** la seconde couche (55) comporte un second agent thérapeutique (60).

13. Dispositif médical selon la revendication 9, comportant de plus une troisième couche de revêtement (65) disposée entre la première couche de revêtement (30) et la seconde couche de revêtement (55), **caractérisé en ce que** la troisième couche de revêtement (65) comprend un second agent thérapeutique de forme particulière (70) et **en ce que** la troisième couche de revêtement (65) est dépourvue de tout matériau polymère.

14. Dispositif médical selon la revendication 1, **caractérisé en ce que** le dispositif médical est un stent.

15. Procédé selon la revendication 2, **caractérisé en ce que** l'étape (b) consiste à :
vaporiser le matériau biologiquement actif sur au moins une partie de la première couche de revêtement (30) ou à plonger le dispositif médical dans le matériau biologiquement actif ou à rouler le dispositif médical dans le matériau biologiquement actif.

16. Procédé selon la revendication 10, comportant de plus la formation d'une troisième couche (65) disposée entre la première couche de revêtement (30) et la seconde couche de revêtement (55), **caractérisé en ce que** la troisième couche de revêtement (65) comprend un second agent thérapeutique de forme particulière (70) et **en ce que** la troisième couche de revêtement (65) est dépourvue de tout matériau polymère.
